# FASCICULE DE BREVET EUROPEEN

(11) **EP 0 521 780 B1**
(45) Date de publication et mention de la délivrance du brevet: **09.12.1998**
(21) Numéro de dépôt: 92401872.4
(22) Date de dépôt: 01.07.1992
(51) Int. Cl.: C07D 209/48, A01N 53/00

(54) **Nouveaux esters pyréthrinoides de l'alcool 1,3,4,5,6,7-hexahydro 1,3-dioxo 2H-isoindol-2-yl méthylique, leur procédé de préparation et leur application comme pesticides**
Pyrethroide Ester des 1,3,4,5,6,7-Hexahydro-1,3-dioxol 2H-isoindol-2-yl Methylalkohols, Verfahren zu ihrer Herstellung und ihre Anwendung als Pestizide
Pyrethroid esters of 1,3,4,5,6,7-hexahydro-1,3-dioxo 2H-isoindol-2-yl methylalcohol, process for their preparation and their application as pesticides

(30) Priorité: 04.07.1991 FR 9108379
(43) Date de publication de la demande: 07.01.1993
(73) Titulaire: ROUSSEL UCLAF, 93230 Romainville (FR)
(72) Inventeur: Babin, Didier, F-78180 Montigny (FR); Benoit, Marc, F-13360 Roquevaire (FR); Demassey, Jacques, F-77144 Montevrain (FR); Demoute, Jean-Pierre, F-93360 Neuilly Plaisance (FR)
(74) Mandataire: Vieillefosse, Jean-Claude

(56) Documents cités:
- EP-A- 41 021
- FR-A- 2 409 261
- GB-A- 1 052 119

## Description

La présente invention concerne de nouveaux esters pyréthrinoïdes de l'alcool 1,3,4,5,6,7-hexahydro 1,3-dioxo-2H-isoindol-2-yl-méthylique, leur procédé de préparation et leur application comme pesticides.

L'invention a pour objet les composés de formule (I) : 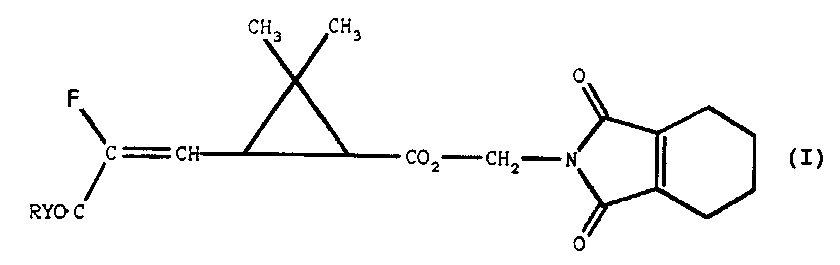 dans laquelle :
- Y représente un atome d'oxygène ou de soufre,
- R représente un radical alkyle linéaire, ramifié ou cyclique, saturé ou insaturé, renfermant jusqu'à 4 atomes de carbone, sous toutes leurs formes stéréoisomères possibles ainsi que leurs mélanges.

Lorsque R représente un radical alkyle saturé, linéaire ou ramifié il s'agit de préférence d'un radical méthyle, éthyle, propyle, isopropyle, butyle, sec-butyle, isobutyle ou tert-butyle.

Lorsque R représente un radical cyclique, il s'agit de préférence d'un radical cyclopropyle ou cyclobutyle, d'un radical alkyle, linéaire ou ramifié, portant un radical cyclique ou d'un radical cyclopropyle substitué par un radical alkyle dont la liaison avec le groupement -COO- est située sur l'un quelconque de ses sommets.

Lorsque R représente un radical alkyle insaturé, il s'agit d'un radical éthylénique, comme par exemple d'un radical vinyle ou d'un radical acétylénique, comme, par exemple, le radical éthynyle ou propynyle.

L'invention a notamment pour objet :
- les composés de formule (I) dans lesquels la copule cyclopropanique est de structure 1Rcis,
- les composés de formule (I) dans lesquels la géométrie de la double liaison vinylique est E,
- les composés de formule (I) dans lesquels Y représente un atome d'oxygène,
et notamment les composés de formule (I) dans lesquels R représente un radical cyclopropyle, et ceux dans lesquels R représente un radical éthyle.

L'invention a plus particulièrement pour objet, les composés de formule (I) dont la préparation est donnée ci-après dans la partie expérimentale.

Les composés de formule (I) présentent d'intéressantes propriétés qui permettent leur utilisation dans la lutte contre les parasites. Il peut s'agir par exemple de la lutte contre les parasites des locaux, les parasites des végétaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des locaux, les parasites des végétaux et les parasites des animaux à sang chaud.

Les composés de formule (I) peuvent être utilisés pour lutter contre les insectes dans les locaux, pour lutter notamment contre les mouches, les moustiques et les blattes.

Les produits de formule (I) peuvent être utilisés également pour lutter contre les insectes dans le domaine agricole, pour lutter par exemple, contre les pucerons, les larves de lépidoptères et les coléoptères. Ils sont utilisés à des doses comprises entre 10 g et 300 g de matière active à l'hectare.

Les composés de formule (I) présentent notamment un très bon pouvoir de knock down.

Il ressort des résultats de tests biologiques ci-après que les produits de formule (I) possèdent également une remarquable activité aphicide.

Les produits de formule (I) peuvent aussi être utilisés pour lutter contre les acariens parasites des végétaux.

Les composés de formule (I) peuvent aussi être utilisés pour lutter contre les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des végétaux, les parasites des locaux et les parasites des animaux à sang chaud, caractérisées en ce qu'elles renferment au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

L'invention a notamment pour objet les compositions insecticides renfermant comme principe actif au moins l'un des produits définis ci-dessus.

Les compositions insecticides selon l'invention contiennent de préférence de 0,005 % à 10 % en poids de matière active.

Selon un mode opératoire avantageux, pour un usage dans les locaux, les compositions insecticides selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco. La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet, les compositions acaricides renfermant comme principe actif au moins un des produits de formule (I) définis ci-dessus.

L'invention a également pour objet les compositions nématicides renfermant comme principe actif au moins un des produits de formule (I) ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables pour pulvérisation foliaire, contenant de 1 à 80 % ou des liquides pour pulvérisation foliaire contenant de 1 à 500 g/l de principe actif. On peut également employer des poudres pour poudrage foliaire contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

On peut indiquer également que les produits de l'invention peuvent être utilisés comme biocides ou comme régulateur de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3,4,5,6-tétrahydrothiophénylidène méthyl) cyclopropanecarboxyliques, par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréo isomères possibles de même que les copules acides et alcools des esters pyréthrinoïdes ci-dessus.

Les associations selon l'invention présentent notamment l'intérêt soit de permettre de combattre, par la polyvalence de leur action, une gamme de parasites plus étendue, soit de manifester, dans certains cas, un effet de synergie.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2,2-1]5-heptène-2,3-dicarboximide, ou le pipéronyl-bis-2-(2'-n-butoxy éthoxy) éthylacétal (ou tropital).

L'invention a donc pour objet les compositions pesticides définies précédemment, caractérisées en ce qu'elles renferment en outre un synergiste des pyréthrinoïdes.

L'invention a également pour objet un procédé de préparation des composés de formule (I), caractérisé en ce que l'on soumet un acide de formule (II) : 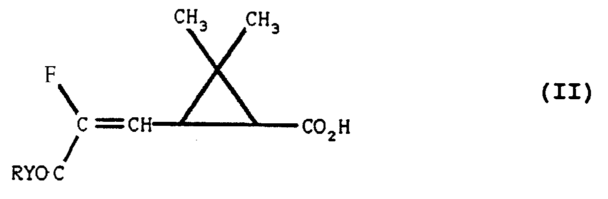 dans laquelle Y et R sont définis comme précédemment, ou un dérivé fonctionnel de cet acide, avec l'alcool de formule (III) : 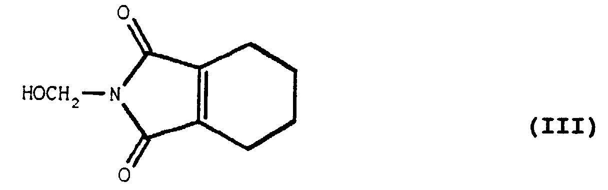 ou un dérivé fonctionnel de cet alcool, pour obtenir le composé de formule (I) correspondant.

Le dérivé fonctionnel d'acide utilisé est de préférence un chlorure d'acide.

Dans un mode de réalisation préféré, on fait réagir l'acide de formule (II) et l'alcool de formule (III) en présence de dicyclohexylcarbodiimide.

Les acides de formule (II) dans lesquels Y représente un atome d'oxygène sont des produits connus (cf EP 50534).

Les acides de formule (II) dans lesquels Y représente un atome de soufre sont des produits connus d'une façon générale qui peuvent être préparés par hydrolyse d'esters connus décrits dans le brevet européen 0108679.

Les exemples suivants illustrent l'invention sans toutefois la limiter.

### EXEMPLE 1 : 1R[1alpha,3alpha(E)] 3-[2-fluoro 3-éthoxy 3-oxo 1-propenyl] 2,2-diméthyl cyclopropanecarboxylate de (1,3,4,5,6,7-hexahydro 1,3-dioxo 2H-isoindol-2-yl) méthyle

On introduit à 0°C, une solution renfermant 1,3 g de dicyclohexylcarbodiimide et 5 ml de chlorure de méthylène, dans un mélange renfermant 980 mg d'alcool (1,3,4,5,6,7-hexahydro 1,3-dioxo 2H-isoindol) méthylique, 1,47 g d'acide 1R[1alpha,3alpha(E)] 3-[2-fluoro 3-éthoxy 3-oxo 1-propényl] 2,2-diméthyl cyclopropanecarboxylique, 10 ml de chlorure de méthylène et 70 mg de diméthylaminopyridine. On agite le milieu réactionnel pendant 16 heures à la température ambiante. On filtre, rince au chlorure de méthylène et concentre à sec. On obtient 2,68 g d'un produit que l'on chromatographie sur silice en éluant avec le mélange hexane-acétate d'éthyle (9-1). On obtient ainsi 2,05 g de produit recherché.
[alpha]_{**D**} = +2 ± 1° (c = 0,9 % CHCl₃).

En opérant comme précédemment à partir des acides (II) appropriés et de l'alcool (III), on a obtenu les produits suivants :

### EXEMPLE 2 : IR-[1alpha,3alpha(E)] 3-[2-fluoro 3-méthoxy 3-oxo 1-propényl] 2,2-diméthyl cyclopropanecarboxylate de (1,3,4,5,6,7-hezahydro 1,3-dioxo 2H-isoindol-2-yl) méthyle,

F=97°C
[alpha]_{**D**} = +3 ± 1° (c = 1 % CHCl₃)

### EXEMPLE 3 : [IR-(1alpha,3alpha)(E) 3-[2-fluoro 3-cyclopropyloxy 3-oxo 1-propényl] 2,2-diméthyl cyclopropanecarboxylate de (1,3,4,5,6,7-hexahydro 1,3-dioxo 2H-isoindol-2-yl) méthyle,

[alpha]_{**D**} = +1°5 ± 2° (c = 0,3 % CHCl₃)

### EXEMPLE 4 : [IR-(1alpha,3alpha) 3-[2-fluoro 3-méthylthio 3-oxo 1-propényl] 2,2-diméthyl cyclopropanecarboxylate de (1,3,4,5,6,7-hexahydro 1,3-dioxo 2H-isoindol-2-yl) méthyle.

[alpha]_{**D**} = -21° ± 2° (c = 0,6 % CHCl₃)

### EXEMPLE 5 : Préparation d'un concentré émulsifiable

On effectue un mélange homogène de :

| | |
|---|---|
| Produit de l'exemple 1 | 1,5 g |
| Tween 80 | 20,0 g |
| Topanol A | 0,1 g |
| Xylène | 78,4 g |

### EXEMPLE 6 : Préparation d'une composition fumigène

On mélange d'une façon homogène :

| | |
|---|---|
| Produit de l'exemple 1 | 0,25 g |
| Poudre de Tabu | 25,00 g |
| Poudre de feuille de cèdre | 40,00 g |
| Poudre de bois de pin | 33,75 g |
| Vert brillant | 0,50 g |
| p-nitrophénol | 0,50 g |

### ACTIVITE PESTICIDE

### Etude de l'effet d'abattage sur mouche domestique

Les insectes tests sont des mouches domestiques femelles âgées de 4 jours. On opère par pulvérisation directe à la concentration de 0,1 g/l en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant, ils sont exprimés en puissance relative par rapport à la bioalléthrine.
Produit de l'exemple 1 = 12,6
Produit de l'exemple 2 = 7,6
Produit de l'exemple 3 = 11,3
Produit de l'exemple 4 = 5,6

## Revendications

1. Les composés de formule (I) : 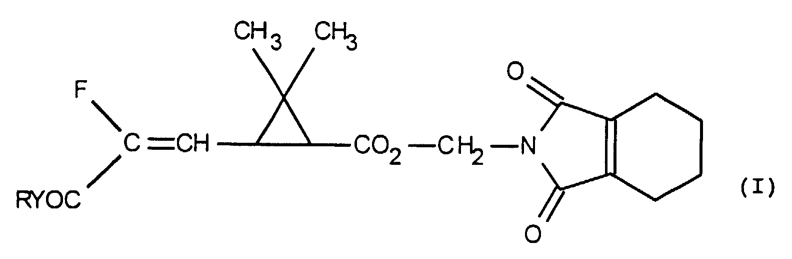 dans laquelle :
- Y représente un atome d'oxygène ou de soufre,
- R représente un radical alkyle linéaire, ramifié ou cyclique, saturé ou insaturé, renfermant jusqu'à 4 atomes de carbone, sous toutes leurs formes stéréoisomères possibles ainsi que leurs mélanges.

2. Les composés de formule (I) tels que définis à la revendication 1 dans lesquels la copule cyclopropanique est de structure 1Rcis.

3. Les composés de formule (I) tels que définis à la revendication 1 ou 2, dans lesquels la géométrie de la double liaison vinylique est E.

4. Les composés de formule (I) tels que définis à la revendication 1, 2 ou 3 dans lesquels Y représente un atome d'oxygène.

5. Les composés de formule (I) tels que définis à l'une quelconque des revendications 1 à 4 dans lesquels R représente un radical éthyle.

6. Les composés de formule (I) tels que définis l'une quelconque des revendications 1 à 4 dans lesquels R représente un radical cyclopropyle.

7. Les composés de formule (I) tels que définis à la revendication 1 dont les noms suivent :
- le 1R-[1alpha,3alpha(E)] 3-[2-fluoro 3-éthoxy 3-oxo 1-propényl] 2,2-diméthyl cyclopropanecarboxylate de (1,3,4,5,6,7-hexahydro 1,3-dioxo 2H-isoindol-2-yl) méthyle,
- le 1R-[1alpha,3alpha(E)] 3-[2-fluoro 3-méthoxy 3-oxo 1-propényl] 2,2-diméthyl cyclopropanecarboxylate de (1,3,4,5,6,7-hexahydro 1,3-dioxo 2H-isoindol-2-yl) méthyle,
- le [1R-(1alpha,3alpha)(E) 3-[2-fluoro 3-cyclopropyloxy 3-oxo 1-propényl] 2,2-diméthyl cyclopropanecarboxylate de (1,3,4,5,6,7-hexahydro 1,3-dioxo 2H-isoindol-2-yl) méthyle,
- le [1R-(1alpha,3alpha)(E) 3-[2-fluoro 3-méthylthio 3-oxo 1-propényl] 2,2-diméthyl cyclopropanecarboxylate de (1,3,4,5,6,7-hexahydro 1,3-dioxo 2H-isoindol-2-yl) méthyle.

8. Les compositions destinées à la lutte contre les parasites des locaux, les parasites des végétaux et les parasites des animaux à sang chaud, caractérisées en ce qu'elles renferment comme principe actif au moins un des produits définis à l'une quelconque des revendications 1 à 7.

9. Les compositions insecticides renfermant comme principe actif au moins l'un des produits définis à l'une quelconque des revendications 1 à 7.

10. Les compositions acaricides renfermant comme principe actif au moins l'un des produits définis à l'une quelconque des revendications 1 à 7.

11. Les compositions nématicides renfermant comme principe actif au moins l'un des produits définis à l'une quelconque des revendications 1 à 7.

12. Les compositions acaricides destinées à la lutte contre les acariens parasites des animaux à sang chaud, notamment contre les tiques et les gales, caractérisées en ce qu'elles renferment comme principe actif, au moins l'un des produits définis à l'une quelconque des revendications 1 à 7.

13. Associations douées d'activité insecticide, acaricide ou nématicide, caractérisées en ce qu'elles contiennent comme matière active, d'une part un au moins des composés de formule générale (I) telle que définie à la revendication 1, et d'autre part, un au moins des esters pyréthrinoïdes choisis dans le groupe constitué par les esters d'alléthrolone, d'alcool 3,4,5,6-tétrahydro phtalimido méthylique, d'alcool 5-benzyl 3-furyl méthylique d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxybenzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

14. Les compositions telles que définies à l'une des revendications 9 à 13, caractérisées en ce qu'elles renferment en outre un synergiste des pyréthrinoïdes.

15. Procédé de préparation des composés de formule (I) tels que définis à l'une quelconque des revendications 9 à 13, caractérisé en ce que l'on soumet un acide de formule (II) : 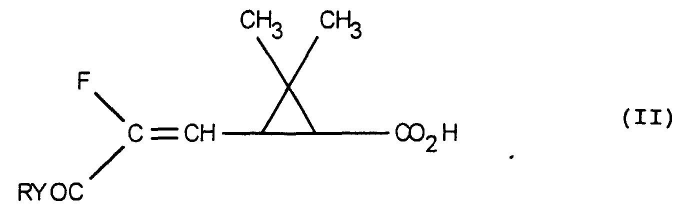 dans laquelle Y et R sont définis comme précédemment, ou un dérivé fonctionnel de cet acide, avec l'alcool de formule (III) : 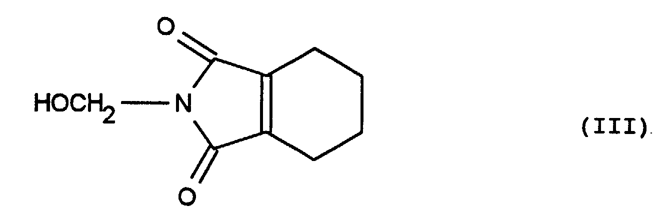 ou un dérivé fonctionnel de cet alcool, pour obtenir le composé de formule (I) correspondant.

## Patentansprüche

1. Verbindungen der Formel (I) 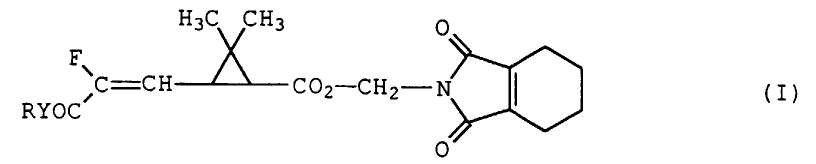 in der
- Y ein Atom von Sauerstoff oder Schwefel darstellt,
- R einen gesättigten oder ungesättigten, geraden, verzweigten oder cyclischen Rest Alkyl mit bis zu 4 Kohlenstoffatomen bedeutet,
in allen ihren möglichen stereoisomeren Formen sowie ihren Mischungen.

2. Verbindungen der Formel (I) wie in Anspruch 1 definiert, worin die Cyclopropan-Kopplung die Struktur lRcis besitzt.

3. Verbindungen der Formel (I) wie in Anspruch 1 oder 2 definiert, worin die Geometrie der vinylischen Doppelbindung E ist.

4. Verbindungen der Formel (I) wie in Anspruch 1, 2 oder 3 definiert, worin Y ein Sauerstoffatom darstellt.

5. Verbindungen der Formel (I) wie in irgendeinem der Ansprüche 1 bis 4 definiert, worin R einen Rest Ethyl darstellt.

6. Verbindungen der Formel (I) wie in irgendeinem der Ansprüche 1 bis 4 definiert, worin R einen Rest Cyclopropyl darstellt.

7. Verbindungen der Formel (I) wie in Anspruch 1 definiert, deren Namen folgen:
- 1R-[1alpha,3alpha(E)]-3-[2-Fluor-3-ethoxy-3-oxo-1-propenyl]-2,2-dimethyl-cyclopropancarbonsäure-(1,3,4,5,6,7-hexahydro-1,3-dioxo-2H-isoindol-2-yl)-methylester,
- 1R-[1alpha,3alpha(E)]-3-[2-Fluor-3-methoxy-3-oxo-1-propenyl]-2,2-dimethyl-cyclopropancarbonsäure-(1,3,4,5,6,7-hexahydro-1,3-dioxo-2H-isoindol-2-yl)-methylester,
- [1R-(1alpha,3alpha) (E)-3-[2-Fluor-3-cyclopropyloxy-3-oxo-1-propenyl]-2,2-dimethyl-cyclopropancarbonsäure-(1,3,4,5,6,7-hexahydro-1,3-dioxo-2H-isoindol-2-yl)-methylester,
- [1R-(1alpha,3alpha) (E)-3- [2-Fluor-3-methylthio-3-oxo-1-propenyl]-2,2-dimethyl-cyclopropancarbonsäure-(1,3,4,5,6,7-hexahydro-1,3-dioxo-2H-isoindol-2-yl)-methylester.

8. Zusammensetzungen zur Bekämpfung von Parasiten in Räumen, Parasiten an Pflanzen und Parasiten an Warmblüter-Tieren, dadurch gekennzeichnet, daß sie als Wirkstoff mindestens eines der in irgendeinem der Ansprüche 1 bis 7 definierten Produkte umfassen.

9. Insektizide Zusammensetzungen, die als Wirkstoff mindestens eines der in irgendeinem der Ansprüche 1 bis 7 definierten Produkte umfassen.

10. Akarizide Zusammensetzungen, die als Wirkstoff mindestens eines der in irgendeinem der Ansprüche 1 bis 7 definierten Produkte umfassen.

11. Nematizide Zusammensetzungen, die als Wirkstoff mindestens eines der in irgendeinem der Ansprüche 1 bis 7 definierten Produkte umfassen.

12. Akarizide Zusammensetzungen zur Bekämpfung von Acariasis-Parasiten an Warmblüter-Tieren, insbesondere von Zecken und Krätzmilben, dadurch gekennzeichnet, daß sie als Wirkstoff mindestens eines der in irgendeinem der Ansprüche 1 bis 7 definierten Produkte umfassen.

13. Assoziationen mit insektizider, akarizider oder nematizider Wirkung, dadurch gekennzeichnet, daß sie als Wirkstoff einerseits mindestens eine der wie in Anspruch 1 definierten Verbindungen der allgemeinen Formel (I) und andererseits mindestens einen Pyrethrinoid-ester umfassen, ausgewählt aus der Gruppe, die gebildet wird durch die Ester von Allethrolon, von 3,4,5,6-Tetrahydrophthalimido-methylalkohol, von 5-Benzyl-3-furyl-methylalkohol, von 3-Phenoxy-benzylalkohol und von alpha-Cyano-3-phenoxy-benzylalkoholen mit Chrysanthemsäuren, durch die Ester von 5-Benzyl-3-furyl-methylalkohol mit 2,2-Dimethyl-3-(2-oxo-3-tetrahydrothiophenyliden-methyl)-cyclopropancarbonsäuren, durch die Ester von 3-Phenoxy-benzylalkohol und von alpha-Cyano-3-phenoxy-benzylalkohol mit 2,2-Dimethyl-3-(2,2-dichlorvinyl)-cyclopropancarbonsäuren, durch die Ester von alpha-Cyano-3-phenoxy-benzylalkoholen mit 2,2-Dimethyl-3-(2,2-dibromvinyl)-cyclopropancarbonsäuren, durch die Ester von 3-Phenoxy-benzylalkohol mit 2-para-Chlorphenyl-2-isopropyl-essigsäuren, durch die Ester von Allethrolon, von 3,4,5,6-Tetrahydro-phthalimido-methylalkohol, von 5-Benzyl-3-furyl-methylalkohol, von 3-Phenoxy-benzylalkohol und von alpha-Cyano-3-phenoxy-benzylalkohol mit 2,2-Dimethyl-3-(1,2,2,2-tetrahaloethyl)-cyclopropancarbonsäuren, worin "halo" ein Atom von Fluor, Chlor oder Brom darstellt, mit der Maßgabe, daß die sauren Kopplungen und die oben genannten Alkohole der Pyrethrinoid-ester in allen ihren möglichen stereoisomeren Formen existieren können.

14. Zusammensetzungen wie in irgendeinem der Ansprüche 9 bis 13 definiert, dadurch gekennzeichnet, daß sie außerdem einen Synergisten der Pyrethrinoide umfassen.

15. Verfahren zur Herstellung der Verbindungen der Formel (I) wie in irgendeinem der Ansprüche 9 bis 13 definiert, dadurch gekennzeichnet, daß man eine Säure der Formel (II) 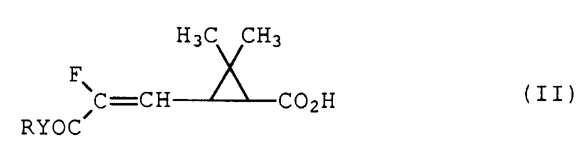 in der Y und R wie vorstehend definiert sind, oder ein funktionelles Derivat dieser Säure mit einem Alkohol der Formel (III) 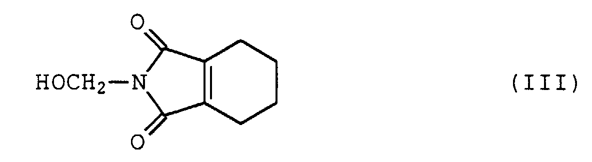 oder einem funktionellen Derivat dieses Alkohols zur Reaktion bringt, um die entsprechende Verbindung der Formel (I) zu erhalten.

## Claims

1. The compounds of formula (I): 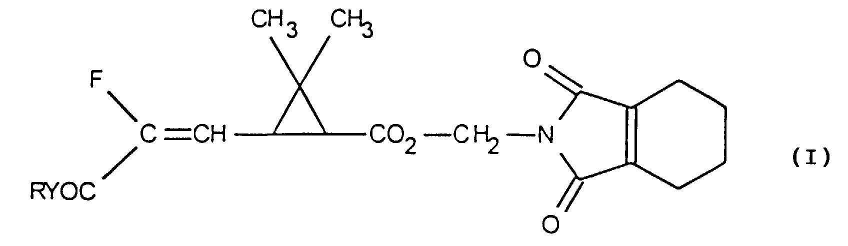 in which:
- Y represents an oxygen or sulphur atom,
- R represents a substituted, saturated or unsaturated, linear, branched or cyclic alkyl radical containing up to 4 carbon atoms, in all their possible stereoisomer forms as well as their mixtures.

2. The compounds of formula (I) as defined in claim 1 in which the cyclopropane copula is of 1Rcis structure.

3. The compounds of formula (I) as defined in claim 1 or 2, in which the geometry of the vinyl double bond is E.

4. The compounds of formula (I) as defined in claim 1, 2 or 3 in which Y represents an oxygen atom.

5. The compounds of formula (I) as defined in any one of claims 1 to 4 in which R represents an ethyl radical.

6. The compounds of formula (I) as defined in any one of claims 1 to 4 in which R represents a cyclopropyl radical.

7. The compounds of formula (I) as-defined in claim 1 the names of which follow:
- 1R-[1alpha,3alpha(E)] 3-[2-fluoro 3-ethoxy 3-oxo 1-propenyl] 2,2-dimethyl cyclopropanecarboxylate of (1,3,4,5,6,7-hexahydro 1,3-dioxo 2H-isoindol-2-yl) methyl,
- 1R-[1alpha,3alpha(E)] 3-[2-fluoro 3-methoxy 3-oxo 1-propenyl] 2,2-dimethyl cyclopropanecarboxylate of (1,3,4,5,6,7-hexahydro 1,3-dioxo 2H-isoindol-2-yl) methyl,
- [1R-(1alpha,3alpha)(E)] 3-[2-fluoro 3-cyclopropyloxy 3-oxo 1-propenyl] 2,2-dimethyl cyclopropanecarboxylate of (1,3,4,5,6,7-hexahydro 1,3-dioxo 2H-isoindol-2-yl) methyl,
- [1R-(1alpha,3alpha)(E)] 3-[2-fluoro 3-methylthio 3-oxo 1-propenyl] 2,2-dimethyl cyclopropanecarboxylate of (1,3,4,5,6,7-hexahydro 1,3-dioxo 2H-isoindol-2-yl) methyl.

8. The compositions intended for combating parasites of premises, parasites of vegetation and parasites of warm-blooded animals, characterized in that they contain as active ingredient at least one of the products defined in any one of claims 1 to 7.

9. The insecticide compositions containing as active ingredient at least one of the products defined in any one of claims 1 to 7.

10. The acaricide compositions containing as active ingredient at least one of the products defined in any one of claims 1 to 7.

11. The nematicide compositions containing as active ingredient at least one of the products defined in any one of claims 1 to 7.

12. The acaricide compositions intended for combating parasitic acaridae of warm-blooded animals, in particular ticks and mange, characterized in that they contain as active ingredient, at least one of the products defined in any one of claims 1 to 7.

13. Combinations endowed with insecticide, acaricide or nematicide activity, characterized in that they contain as active ingredient, on the one hand at least one of compounds of general formula (I) as defined in claim 1, and on the other hand, at least one of the pyrethrinoid esters chosen from the group constituted by the esters of allethrolone, of 3,4,5,6-tetrahydrophthalimidomethyl alcohol, of 5-benzyl 3-furyl methyl alcohol, of 3-phenoxybenzyl alcohol and of alpha cyano 3-phenoxybenzyl alcohols with chrysanthemic acids, by the esters of 5-benzyl-3-furyl methyl alcohol with 2,2-dimethyl 3-(2-oxo 3-3,4,5,6-tetrahydrothio phenylidenemethyl) cyclopropanecarboxylic acids, by the esters of 3-phenoxybenzyl alcohol and of alpha cyano 3-phenoxybenzyl alcohols with 2,2-dimethyl 3-(2,2-dichlorovinyl) cyclopropanecarboxylic acids, by the esters of alpha cyano 3-phenoxy benzyl alcohols with 2,2-dimethyl 3-(2,2-dibromovinyl) cyclopropanecarboxylic acids, by the esters of 3-phenoxybenzyl alcohols with 2-parachlorophenyl 2-isopropyl acetic acids, by the esters of allethrolone, of 3,4,5,6-tetrahydrophthalimidomethyl alcohol, of 5-benzyl 3-furyl methyl alcohol, of 3-phenoxybenzyl alcohol, and of alpha cyano 3-phenoxybenzyl alcohols with 2,2-dimethyl 3-(1,2,2,2-tetrahaloethyl) cyclopropanecarboxylic acids, in which "halo" represents a fluorine, chlorine or bromine atom, it being understood that the acid and alcohol copulas of the above pyrethrinoid esters can exist in all their possible stereoisomer forms.

14. The compositions as defined in any one of claims 9 to 13, characterized in that they contain in addition a pyrethrinoid synergist.

15. Preparation process for the compounds of formula (I) as defined in any one of claims 9 to 13, characterized in that an acid of formula (II): 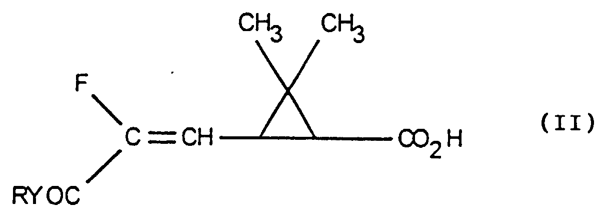 in which Y and R are defined as previously, or a functional derivative of this acid, is subjected to the alcohol of formula (III): 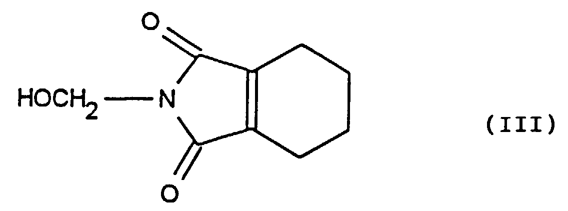 or a functional derivative of this alcohol, in order to obtain the corresponding compound of formula (I).
